# FASCICULE DE BREVET EUROPEEN

(11) **EP 3 765 595 B1**
(45) Date de publication et mention de la délivrance du brevet: **01.12.2021**
(21) Numéro de dépôt: 19716966.7
(22) Date de dépôt: 12.03.2019
(51) Int. Cl.: C12M 3/00, A61F 2/14, C12M 1/12, C12N 5/079

(54) **PROCEDE ET DISPOSITIF POUR LA PREPARATION D'UN IMPLANT ISSU D'UNE CULTURE DE CELLULES SOUCHES**
VERFAHREN UND VORRICHTUNG ZUR HERSTELLUNG EINES AUS EINER STAMMZELLKULTUR GEWONNENEN IMPLANTATS
METHOD AND DEVICE FOR PREPARING AN IMPLANT OBTAINED FROM A CULTURE OF STEM CELLS

(30) Priorité: 12.03.2018 FR 1852114
(43) Date de publication de la demande: 20.01.2021
(73) Titulaire: Centre d'Etude des Cellules Souches (CECS), 91100 Corbeil Essonnes (FR)
(72) Inventeur: BEN M'BAREK, Karim, 94000 Creteil (FR); HABELER, Walter, 75012 Paris (FR); MONVILLE, Christelle, 94500 Champigny sur Marne (FR)
(74) Mandataire: Grosset-Fournier, Chantal Catherine
(86) Numéro de dépôt international: PCT/FR2019/050529
(87) Numéro de publication internationale: WO 2019/175497

(56) Documents cités:
- EP-A1- 2 671 539
- WO-A1-94/21204
- WO-A1-2017/096207
- DE-C1- 3 938 632
- NISTOR G ET AL: "Three-dimensional early retinal progenitor 3D tissue constructs derived from human embryonic stem cells", JOURNAL OF NEUROSCIENCE METHODS, ELSEVIER SCIENCE PUBLISHER B.V., AMSTERDAM, NL, vol. 190, no. 1, 30 juin 2010 (2010-06-30) , pages 63-70, XP027106839, ISSN: 0165-0270 [extrait le 2010-05-04]

## Description

La présente invention concerne la préparation d'un greffon implantable, obtenu à partir d'une culture de cellules souches, notamment des cellules souches de mammifères ou de tout autre type permettant d'obtenir des cellules de l'œil. La présente invention s'applique notamment au traitement de maladies de l'épithélium pigmentaire rétinien et de la rétine neurale.

La rétine est la couche sensorielle qui tapisse la face postérieure de l'œil, reçoit l'image formée par la lentille, traduit cette image en impulsions neuronales qui sont transmises au cerveau par le nerf optique. La rétine est un tissu neuronal qui interagit de façon critique avec l'épithélium pigmentaire rétinien qui lui est nécessaire à sa survie et son bon fonctionnement. La macula constituant la partie centrale de la rétine, est enrichie en photorécepteurs capables de percevoir les différentes couleurs et les détails visuels fins. La macula est donc cruciale notamment pour la reconnaissance faciale et la lecture.

De nombreuses maladies peuvent affecter l'épithélium pigmentaire rétinien ou la rétine neurale et entrainer une altération de la vision pouvant conduire à la cécité. Parmi ces maladies, on peut citer en particulier les dégénérescences maculaires héréditaires ou liées à l'âge, les dystrophies maculaires, et les rétinites pigmentaires. L'épithélium rétinien ou la rétine neurale peut également présenter des lésions d'origine traumatique ou infectieuse. Des recherches récentes semblent démontrer que les lésions de la rétine peuvent être retardées, arrêtées, voire être réparées par la transplantation de cellules d'épithélium pigmentaire rétinien ou de cellules rétiniennes dans l'espace sous-rétinien, ce qui peut prévenir la réduction des capacités visuelles provoquée par ces maladies ou les améliorer dans le cas de greffes de cellules de la rétine neurale.

On a proposé diverses formes de greffes ou de techniques de transplantation pour tenter de préserver la rétine. Plus récemment, des résultats prometteurs ont été obtenus en transplantant une culture de cellules d'épithélium pigmentaire rétinien obtenues par différenciation de cellules souches pluripotentes, les cellules étant cultivées sur une membrane. Par exemple, Nistor et al. (Journal of Neuroscience Methods, vol. 190, no. 1, 30.06.2010) ont décrit un procédé de préparation d'un implant comprenant une culture de cellules sur une membrane.

Pour préparer un implant à partir d'une telle membrane, la membrane doit être retirée d'un support de culture, et être recouverte des deux côtés d'une couche de gélatine. A cet effet, la couche de gélatine est préalablement amincie et uniformisée en lui retirant une couche supérieure à l'aide d'une machine de découpe adaptée, appelée "vibratome". La membrane est ensuite disposée sur la couche de gélatine amincie. La couche de gélatine est à nouveau amincie à l'épaisseur souhaitée en lui retirant une couche inférieure à l'aide de la machine de découpe. Finalement, une goutte de gélatine liquide (à 37°C + ou - 10°C) est versée sur les cellules cultivées sur la membrane, la gélatine étant polymérisée à froid. La membrane qui est ainsi disposée entre deux couches de gélatine polymérisée, est alors conservée et transportée telle qu'elle sur le site d'utilisation. Au moment de la transplantation, la membrane doit être découpée manuellement sensiblement à la forme souhaitée, généralement sous microscope. La transplantation est généralement effectuée à l'aide d'une seringue avec une aiguille ayant un diamètre inférieur aux dimensions de l'implant. Il en résulte que l'implant traverse l'aiguille enroulé sur lui-même.

Il s'avère que ce procédé de préparation présente une grande complexité, nécessitant de nombreuses manipulations manuelles exercées directement sur la membrane, sans que celle-ci soit protégée. Il en résulte de nombreux risques d'altération et d'infection pour la culture. Il s'avère également que les couches de gélatine de chaque côté de la membrane présentent des épaisseurs variables, qui sont en outre non uniformes. En effet, la précision de la machine de découpe s'avère insuffisante et le procédé employé pour former la couche de gélatine supérieure conduit généralement à la réalisation d'une couche bombée. La machine de découpe s'avère également encombrante. De telles variations d'épaisseur entrainent des risques que l'implant reste enroulé sur lui-même lorsqu'il sort de l'aiguille d'injection. Dans le cas de cellules d'épithélium pigmentaire rétinien, l'implant doit être inséré sous la rétine. Il faut donc éviter qu'il soit trop épais lors de son positionnement sous la rétine. Par ailleurs, comme la membrane est séparée de son support de culture, il est difficile de visualiser de quel côté de la membrane se trouve la culture. Il en résulte des risques importants que l'implant soit positionné du mauvais côté à l'issue de l'opération de transplantation.

Il est donc souhaitable de prévoir un procédé simplifié pour réaliser un tel implant, en éliminant les risques d'altération et de contamination de la culture. Il est également souhaitable que l'implant présente une épaisseur maitrisée. Il peut être également souhaitable que l'implant présente des dimensions également maîtrisées, en particulier non liées à des opérations manuelles. Il peut être également souhaitable d'éliminer les erreurs de positionnement de l'implant au moment de la transplantation.

Des modes de réalisation concernent un procédé de préparation d'un implant comprenant une culture de cellules sur une membrane, le procédé comprenant des étapes consistant à : fixer la membrane sur un support, disposer le support dans un logement d'un boitier ménageant deux espaces ayant des hauteurs ajustées, au-dessus et en dessous de la membrane, injecter dans les espaces un liquide apte à se transformer en gel à une température de transport inférieure à une température d'injection du liquide, et porter le boitier à la température de transport, de manière à former un implant comprenant la membrane et deux couches de gel ayant des épaisseurs ajustées, respectivement sur deux faces opposées de la membrane.

Selon un mode de réalisation, le procédé comprend une étape de découpe de l'implant dans la membrane et les deux couches de gel, la membrane étant fixée au support et disposée dans le logement.

Selon un mode de réalisation, le procédé comprend une étape de fermeture du logement du boitier recevant la membrane par un couvercle d'extraction, le couvercle d'extraction étant configuré pour recevoir et guider un outil de découpe configuré pour découper l'implant dans la membrane et les deux couches de gel.

Selon un mode de réalisation, l'implant présente une forme dissymétrique.

Selon un mode de réalisation, les espaces au-dessus et en dessous de la membrane sont formés entre le fond du logement et un couvercle de préparation adapté à refermer le logement.

Selon un mode de réalisation, le procédé comprend une étape de remplacement du couvercle de préparation par un couvercle de transport refermant le logement de manière étanche, lorsque le liquide injecté dans le logement s'est polymérisé en gel.

Selon un mode de réalisation, les liquides injectés dans les espaces supérieur et inférieur sont différents, de manière à ce que la couche de gel supérieure soit moins rigide que la couche de gel inférieure.

Selon un mode de réalisation, les cellules disposées sur la membrane sont dérivées de cellules souches pluripotentes, de cellules souches adultes multipotentes ou bien sont issues de cultures primaires ou de lignées cellulaires, ou bien correspondent à un type cellulaire de la zone d'implantation, ou bien sont des cellules épithéliales simples ou de différents sous-types, tels que des photorécepteurs, des cellules ganglionnaires, des cellules bipolaires, des cellules amacrines, des cellules de l'épithélium pigmentaire rétinien, des cellules endothéliales. Les cellules souches pluripotentes humaines utilisées pour la mise en œuvre de l'invention ne nécessitent pas la destruction d'embryon humain.

Des modes de réalisation peuvent également concerner un dispositif de préparation d'un implant à partir d'une culture de cellules sur une membrane, le dispositif comprenant : un support maintenant la membrane, un boitier comportant un logement pour recevoir le support associé à la membrane, le logement ménageant deux espaces ayant des hauteurs ajustées, au-dessus et en dessous de la membrane, et un orifice de remplissage pour injecter un liquide dans les deux espaces, le liquide étant apte à se transformer en gel à une température de transport inférieure à une température d'injection du liquide, le dispositif étant configuré pour mettre en œuvre le procédé selon l'invention.

Selon un mode de réalisation, le dispositif comprend un couvercle de préparation configuré pour refermer le logement avec le support, le couvercle de préparation délimitant avec la membrane l'espace supérieur, l'espace inférieur étant délimité par le fond du logement et la membrane, le couvercle de préparation comprenant un orifice de remplissage débouchant dans l'espace supérieur, le boitier comprenant un orifice de remplissage débouchant dans l'espace inférieur.

Selon un mode de réalisation, le dispositif comprend un couvercle de transport prévu pour refermer le logement de manière étanche.

Selon un mode de réalisation, le dispositif comprend un couvercle d'extraction prévu pour refermer le logement et comportant un conduit pour le passage d'un outil de découpe configuré pour découper un morceau de la membrane incluant des cellules cultivées, le morceau de membrane découpé formant l'implant.

Selon un mode de réalisation, le dispositif comprend au moins l'une des caractéristiques suivantes : les deux espaces présentent une hauteur comprise entre 0,05 et 0,2 mm, ou bien entre 0,13 et 0,17 mm, l'implant présente une surface comprise entre 10 et 20 mm². la membrane est une membrane amniotique, ou une membrane de Bruch, ou une membrane basale, ou une matrice de fibres protéiques telles que des fibres de collagène et/ou de fibrine ou une membrane en un matériau synthétique biocompatible, biodégradable ou non, le liquide se transformant en gel est un hydrogel naturel ou synthétique biocompatible, qui est liquide à la température intérieure du corps humain, le support de membrane présente une forme tronconique comportant une grande ouverture et une petite ouverture, la membrane étant fixée sur le support de manière à obturer la petite ouverture, la culture de cellules étant réalisée sur une face de la membrane disposée à l'intérieur du support, l'outil de découpe est configuré pour découper un morceau de membrane de forme dissymétrique.

Des modes de réalisation peuvent également concerner une utilisation du dispositif précédemment défini, pour réaliser un implant de cellules cultivées sur une membrane.

Des exemples de réalisation de l'invention seront décrits dans ce qui suit, à titre non limitatif en relation avec les figures jointes parmi lesquelles :
les figures 1 et 2 sont des vues en perspective et en vue axiale éclatée d'un dispositif support de culture de cellules, selon un mode de réalisation,
les figures 3 à 5 sont, respectivement une vue de dessus, une vue de profil et une vue de dessous d'un dispositif de manipulation d'une culture de cellules, selon un mode de réalisation,
les figures 6 et 7 sont, respectivement une vue de dessus, une vue de profil d'un couvercle de préparation d'un implant, adapté pour se fixer sur le dispositif de manipulation, selon un mode de réalisation,
la figure 8 est une vue de profil du couvercle de préparation, en coupe suivant un plan BB montré sur la figure 6,
les figures 9 et 10 sont des vues de profil, respectivement éclatée et non éclatée, du dispositif de manipulation montré en coupe suivant le plan AA indiqué sur la figure 3, associé au couvercle de préparation montré en coupe suivant le plan CC indiqué sur la figure 6,
la figure 10A est une vue de profil en coupe détaillée d'une partie du dispositif de manipulation muni du couvercle de préparation,
la figure 11 est une vue de dessus d'un couvercle de transport, adapté pour se fixer sur le dispositif de manipulation, selon un mode de réalisation,
la figure 12 est une vue de profil du couvercle de transport, en coupe suivant le plan DD montré sur la figure 11,
la figure 13 est une vue du dispositif de manipulation en coupe suivant le plan AA montré sur la figure 3, associé au couvercle de transport en coupe suivant le plan DD montré sur la figure 11,
la figure 14 est une vue de dessus d'un couvercle d'extraction de l'implant, selon un mode de réalisation,
les figures 15 et 16 sont des vues de profil en coupe, respectivement éclatée et non éclatée, du dispositif de manipulation muni du couvercle d'extraction, et d'un dispositif de découpe, selon un mode de réalisation, le couvercle d'extraction figurant en coupe suivant le plan EE montré sur la figure 14, le dispositif de préparation figurant en coupe suivant le plan AA montré sur la figure 3, et le dispositif de découpe figurant en coupe suivant le plan FF montré sur la figure 17,
la figure 17 est une vue axiale détaillée de dessous de l'extrémité inférieure du dispositif de découpe et d'une partie du couvercle d'extraction,
la figure 18 est une vue schématique en coupe d'un implant obtenu par le procédé selon un mode de réalisation.

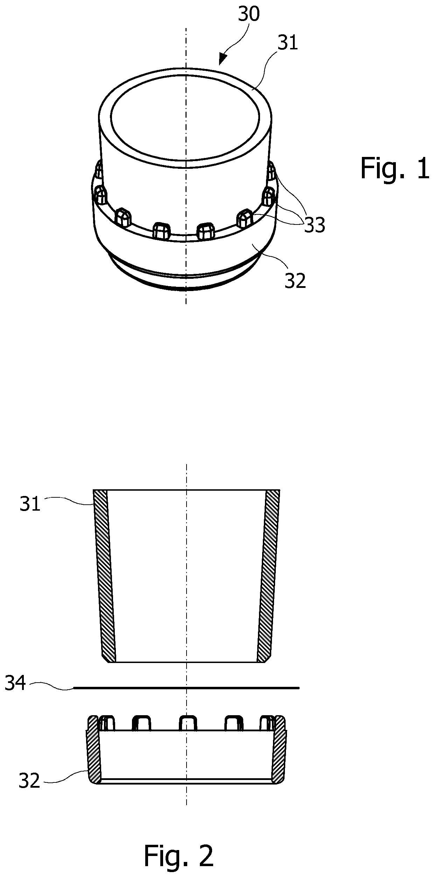

Les figures 1 et 2 représentent un dispositif 30 supportant une culture de cellules, selon un mode de réalisation. Le dispositif support 30 comprend un élément tubulaire 31, de forme tronconique, par exemple à section circulaire, sur lequel vient s'engager une couronne 32. L'élément tubulaire 31 présente ainsi, respectivement à ses deux extrémités, deux ouvertures ayant des diamètres ou largeurs différentes, l'ouverture de plus petite largeur étant présentée vers le bas sur les figures 1 et 2.

La couronne 32 présente également une forme tronconique dont la section présente une forme identique à celle de l'élément tubulaire 31. Le bord de l'ouverture de plus grande largeur de la couronne 32 peut comprendre des picots 33 s'étendant axialement. Les picots permettent de distinguer plus facilement les ouvertures de la couronne 32 pour engager celle-ci sur l'élément tubulaire 31 dans le sens correct.

La largeur de la couronne 32 est telle que cette dernière peut coulisser seulement le long d'une partie de l'élément tubulaire 31, à partir de l'ouverture de plus petite largeur de l'élément tubulaire 31. Les cellules sont cultivées sur une membrane 34 qui est fixée au dispositif support 30, en étant pincée entre l'élément tubulaire 31 et la couronne 32. Les cellules sur la membrane 34 se trouvent ainsi dans l'élément tubulaire 31, l'ouverture de plus petite largeur de l'élément tubulaire étant fermée par la membrane 34. La culture de cellules peut être réalisée à la suite de la fixation de la membrane 34 à l'extrémité de l'élément tubulaire 31.

Si dans l'exemple des figures 1 et 2, le dispositif support présente une section de forme circulaire, toute autre forme de section peut être envisagée, comme une forme carrée, polygonale, rectangulaire, etc.

La membrane 34 peut présenter une épaisseur comprise entre 50 et 100 µm, et être réalisée en un matériau naturel ou synthétique. Ainsi la membrane peut être réalisée à partir d'une membrane amniotique, d'une membrane de Bruch, d'une membrane basale, ou d'une matrice de fibres protéiques telles que des fibres de collagène et/ou de fibrine. Elle peut également être réalisée en un matériau synthétique biocompatible tel qu'un polymère biodégradable. Il importe que les cellules de la culture adhèrent à la membrane choisie.

Selon un exemple de réalisation, les cellules de la culture sont des cellules d'épithélium pigmentaire rétinien obtenues à partir de cellules souches pluripotentes humaines disposées sur une membrane amniotique dénudée, puis cultivées durant quatre semaines.

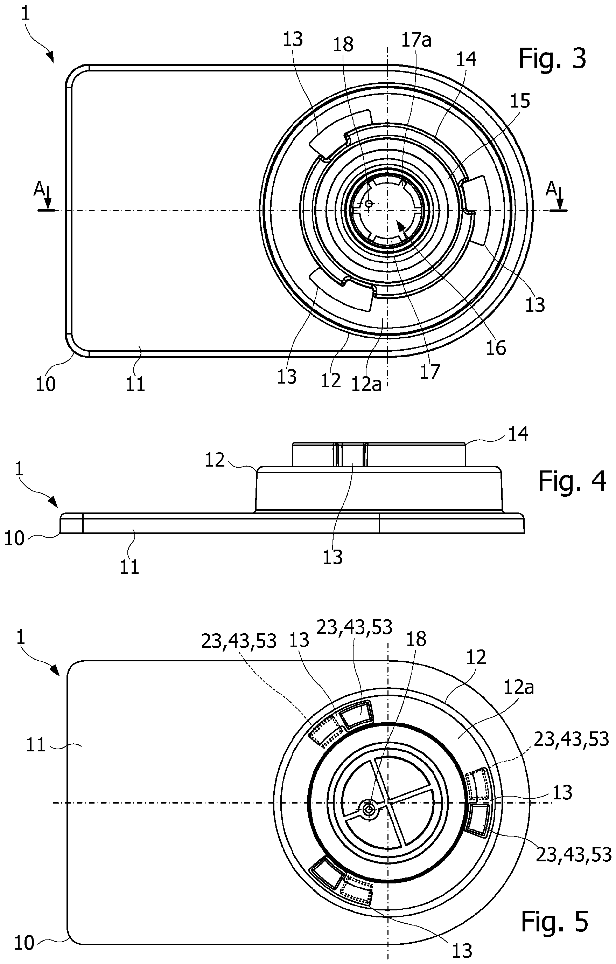

Les figures 3 à 5 représentent un dispositif de manipulation 10 d'une culture de cellules, selon un mode de réalisation. Le dispositif de manipulation 10 comprend un logement 16 prévu pour recevoir le dispositif support 30 et la membrane 34, et des moyens de fermeture 15 et de fixation 13 d'un couvercle permettant de refermer le logement 16, notamment lorsque ce dernier contient le dispositif support 30. Le dispositif de manipulation 10 comporte également un orifice de remplissage 18 réalisé dans le fond du logement 16. Le fond du logement 16 présente également un rebord annulaire 17.

Dans l'exemple des figures 3 à 5, le dispositif de manipulation 10 comprend un plateau 11 solidaire du logement 16. Le logement 16 présente une forme cylindrique comportant un bord externe 12 de forme tubulaire, un bord supérieur 12a plan, de forme annulaire, parallèle au plateau 11, et une nervure annulaire 14 prolongeant axialement le bord supérieur plan 12a. Le logement 16 comporte également une gorge annulaire 15 à l'intérieur de la nervure 14, la gorge annulaire entourant le logement 16.

Dans l'exemple des figures 3 à 5, les moyens de fixation 13 comportent des ouvertures 13 ayant la forme d'arcs annulaires, réalisées dans le bord supérieur plan 12a, et comportant chacune deux parties de largeurs différentes.

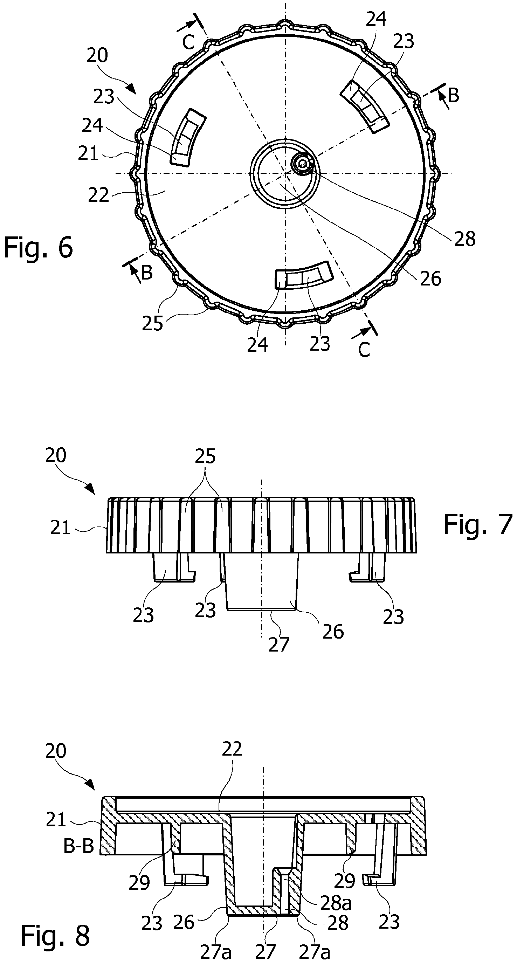

Les figures 6 à 8 représentent un couvercle 20 de préparation d'un implant, adapté pour se fixer sur le dispositif de manipulation 10, selon un mode de réalisation. Le couvercle de préparation 20 comprend un plateau supérieur 22 de forme annulaire, prolongé à l'extérieur par une couronne extérieure 21, et à l'intérieur, par une extension tubulaire 26, s'étendant coaxialement du côté de la face inférieure du plateau supérieur 22. Le couvercle de préparation 20 comprend également des moyens de fixation 23 s'étendant du côté inférieur du couvercle et conformés pour coopérer avec les moyens de fixation 13 du dispositif de manipulation 10. L'extension tubulaire 26 comporte un fond 27 muni d'un orifice de remplissage 28, s'étendant parallèlement au plateau supérieur 22. Le couvercle de préparation 20 comprend également une nervure annulaire 29 s'étendant du côté inférieur du couvercle, et prévue pour coopérer avec la gorge annulaire 15 formée dans le dispositif de manipulation 10.

Dans l'exemple des figures 6 à 8, les moyens de fixation 23 présentent la forme de crochets prévus pour s'engager simultanément dans la partie la plus large des ouvertures 13. Les crochets 23 comportent une partie axiale prolongée par une partie radiale prévue pour se bloquer dans la partie la plus étroite des ouvertures 13 par un mouvement de rotation du couvercle de préparation 20 autour de son axe par rapport au dispositif de manipulation 10. La figure 5 montre les crochets 23 en traits pleins engagés dans la partie la plus large des ouvertures 13, et en traits interrompus en position de verrouillage, engagés dans la partie la plus étroite des ouvertures 13. Pour faciliter l'entrainement en rotation par la main du couvercle 20, la couronne extérieure 21 peut être munie de crans 25. Des ouvertures 24 peuvent être formées dans le plateau supérieur 22, à l'aplomb des parties radiales des crochets 23 pour des raisons de fabrication. L'orifice 28 peut être prolongé à l'intérieur de l'extension tubulaire 26 par une conduite 28a en forme d'entonnoir.

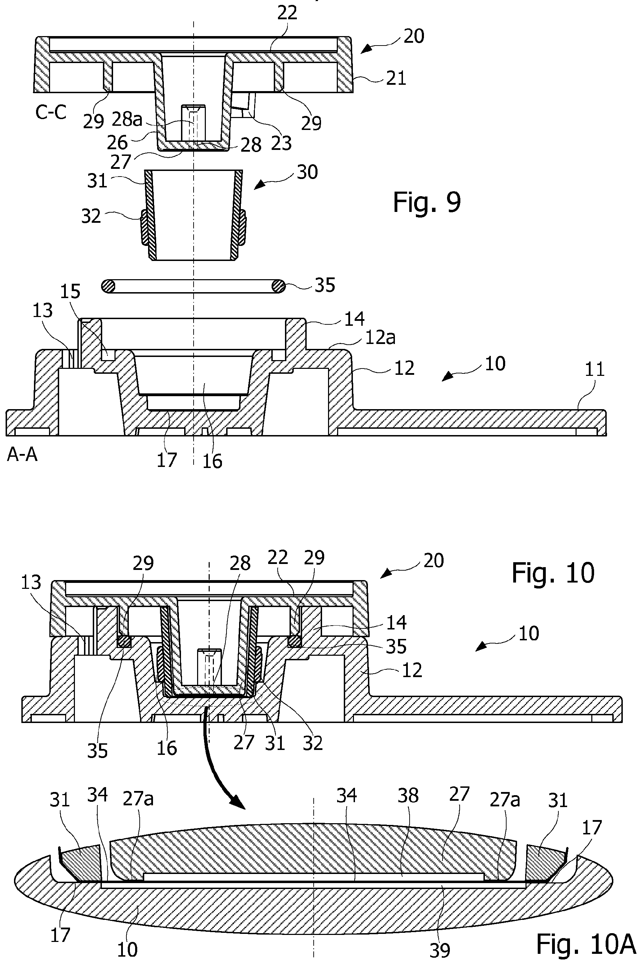

Les figures 9 et 10 représentent l'ensemble du dispositif de manipulation 10 associé au couvercle de préparation 20 et renfermant le dispositif support 30. Il apparaît sur ces figures que lorsque le couvercle 20 est verrouillé sur le dispositif de manipulation 10, l'ensemble du dispositif support 30 peut être enfermé dans le logement 16. L'étanchéité de la fermeture du logement 16 est assurée grâce à un joint torique 35 disposé dans la gorge annulaire 15, le joint 35 étant comprimé par la nervure annulaire 29 formée sous le couvercle 20.

La figure 10A montre en détail le fond du logement 16, avec l'extrémité inférieure 27 de l'extension tubulaire 26, l'extrémité inférieure du dispositif support 30 et la membrane 34 supportant la culture de cellules. Il apparaît sur la figure 10A que le rebord annulaire 17 formé au fond du logement 16 permet de maintenir le dispositif support 30, et donc la membrane 34, à distance du fond du logement 16, délimitant ainsi entre le fond du logement 16 et la membrane un espace vide 39 en forme de disque dans lequel débouche l'orifice de remplissage 18.

Par ailleurs, l'extension tubulaire 26 présente une longueur telle que lorsque le couvercle 20 est verrouillé sur le dispositif de manipulation 10, l'extrémité inférieure 27 de l'extension tubulaire 26 vient en contact avec la face supérieure de la membrane 34. L'extrémité inférieure 27 de l'extension tubulaire 26 comporte un rebord annulaire 27a, ménageant entre l'extrémité inférieure 27 de l'extension tubulaire 26 et la membrane 34 un espace vide 38 en forme de disque dans lequel débouche l'orifice de remplissage 28.

Selon un mode de réalisation, les espaces vides 38, 39 qui présentent une hauteur prédéfinie, sont remplis par une substance, telle qu'un hydrogel naturel ou synthétique biocompatible, présentant un état liquide à la température de l'intérieur du corps humain, soit 37°C, et qui se polymérise en gel à une température inférieure.

Selon un exemple de réalisation, l'un des espaces 38, 39, est rempli de la substance sous forme liquide. Le dispositif de manipulation 10 est ensuite refroidi pour que le liquide se polymérise en gel. L'autre espace 38, 39 est rempli de la substance sous forme liquide et le dispositif de manipulation 10 est à nouveau refroidi. Lors du remplissage de l'espace inférieur 39, par exemple à l'aide d'une micropipette, par l'orifice 18, l'air initialement présent dans cet espace s'évacue par des encoches 17a formées dans le rebord annulaire 17 (figure 3). Lors du remplissage de l'espace supérieur 38 par l'orifice 28, l'air présent dans cet espace s'évacue par l'espace entre le dispositif support 30 et l'extension tubulaire 26.

Ainsi, le dispositif de manipulation 10 combiné au couvercle de préparation 20 permet de préparer un implant comprenant la membrane 34 supportant les cellules à transplanter et couverte de chaque côté d'une couche de gel. Cette opération ne nécessite pas de séparer la membrane 34 de son support 30. Les épaisseurs de gel de chaque côté de la membrane sont fixes et définies de manière à ce que l'implant puisse être enroulé sur lui-même et ainsi passer par l'aiguille d'une seringue utilisée pour la transplantation, et présentant en sortie un diamètre de 1,8 à 2,2 mm. Les épaisseurs de gel de chaque côté de la membrane 34 sont également choisies pour que l'implant retrouve une forme sensiblement plane, naturellement sans manipulation, à la sortie de l'aiguille de la seringue. A l'issue de la transplantation, la substance à l'état de gel avant et durant la transplantation, passe progressivement à l'état liquide et se mélange avec les fluides corporels.

Selon un mode de réalisation, l'espace inférieur 39 est rempli de la substance en premier, puis l'espace supérieur 38 est rempli avant que la substance injectée dans l'espace inférieur 39 soit totalement polymérisée. La quantité de la substance injectée par les deux orifices 18, 28 peut être ajustée de manière à éviter que la membrane 34 se bombe. La concentration de la substance injectée dans l'espace supérieur 38 peut être moindre que celle injectée dans l'espace inférieur 39, afin que l'implant soit moins rigide et donc puisse se courber plus facilement vers le côté de la membrane 34 supportant la culture que vers l'autre côté.

Selon un exemple de réalisation, la substance utilisée pour remplir les espaces 38, 39, est la gélatine qui est à l'état de gel à une température inférieure à 7°C et à l'état liquide à 37°C. La hauteur des espaces 38, 39, c'est-à-dire l'épaisseur de la couche de gel de chaque côté de la membrane 34 est comprise entre 0,05 et 0,2 mm, et de préférence comprise entre 0,13 et 0,17 mm. Le diamètre des orifices 18, 28 peut être de 1 mm (à + ou - 20% près). La gélatine injectée dans l'espace inférieur 39 est diluée à une concentration de 8% (+ ou -2%) dans du sérum physiologique, et est laissée polymérisée pendant 3 minutes à une température de 4°C (+ ou -3°C), la polymérisation complète étant obtenue au bout de 5 minutes à ces températures. La gélatine injectée dans l'espace supérieur 38 est diluée à une concentration de 5% (+ ou -2%) dans du sérum physiologique. L'ensemble du dispositif de manipulation 10 et du couvercle 20, incluant la culture est ensuite maintenu à une température inférieure à 7°C pendant plus de 5 minutes.

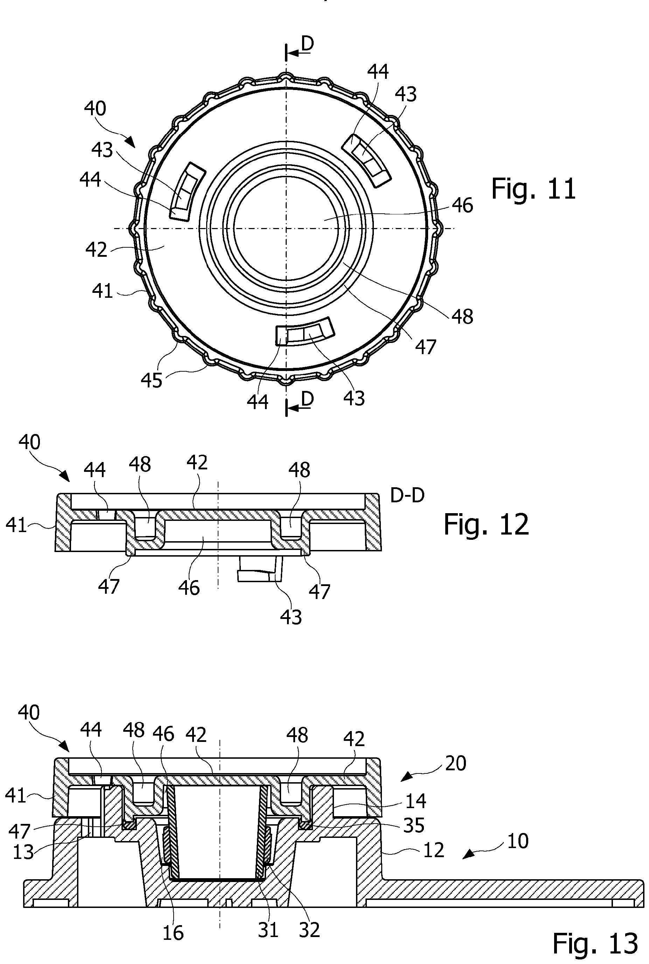

Les figures 11 et 12 représentent un couvercle de transport 40, adapté pour se fixer sur le dispositif de manipulation 10, selon un mode de réalisation. La figure 13 montre le couvercle de transport 40 fixé sur le dispositif de manipulation 10. Une fois que la substance requise a été injectée dans les espaces 38, 39 et s'est polymérisée en gel par réfrigération, le couvercle de préparation 20 est retiré et remplacé par le couvercle de transport 40. Le couvercle de transport 40 associé au support 30 et au dispositif de manipulation 10 assure le maintien de l'implant durant le transport afin de prévenir toute dissociation des couches de gélatine, notamment sous l'effet de vibrations.

Un liquide adapté peut être préalablement utilisé pour remplir le volume au-dessus de la couche de gel dans l'élément tubulaire 31 du support 30, notamment afin de maintenir la couche en gel en milieu humide. Ce liquide peut être par exemple un liquide utilisé pour le transport de cornée humaine tel que le STEM ALPHA. 1 commercialisé par la société STEM ALPHA. Ainsi le dispositif de manipulation 10 associé au couvercle de transport permet de transporter l'implant jusqu'au site où il doit être transplanté et de le conserver à froid (à une température inférieure à 10°C) jusqu'au moment de la transplantation.

Le couvercle de transport 40 présente une forme similaire à celle du couvercle de préparation 20, mais sans extension tubulaire 26. Ainsi, le couvercle 40 comprend un plateau supérieur 42 en forme de disque, prolongé à l'extérieur par une couronne extérieure 41. Le couvercle de transport 40 comprend également des moyens de fixation 43 s'étendant du côté inférieur du couvercle et conformés pour coopérer avec les moyens de fixation 13 du dispositif de manipulation 10. Le couvercle de transport 40 comprend également une nervure annulaire 47 s'étendant du côté inférieur du couvercle, et prévue pour coopérer avec le joint torique 35 disposé dans la gorge annulaire 15 formée dans le dispositif de manipulation 10, pour assurer l'étanchéité de la fermeture du logement 16. Ainsi, le logement 16 est fermé de manière étanche, l'orifice de remplissage 18 étant obturé par le gel.

Dans l'exemple des figures 11 à 13, les moyens de fixation 43 présentent la forme de crochets prévus pour s'engager simultanément dans la partie la plus large des ouvertures 13. Les crochets 43 comportent une partie axiale prolongée par une partie radiale prévue pour se bloquer dans la partie la plus étroite des ouvertures 13 par un mouvement de rotation du couvercle de transport 40 autour de son axe par rapport au dispositif de manipulation 10. Pour faciliter l'entrainement en rotation par la main du couvercle 40, la couronne extérieure 41 peut être munie de crans 45. Des ouvertures 44 peuvent être formées dans le plateau supérieur 42, à l'aplomb des parties radiales des crochets 43 pour des raisons de fabrication. Le plateau 42 peut comprendre une gorge annulaire 48 formant côté intérieur du couvercle un rebord annulaire assurant le maintien de la partie supérieure du support 30.

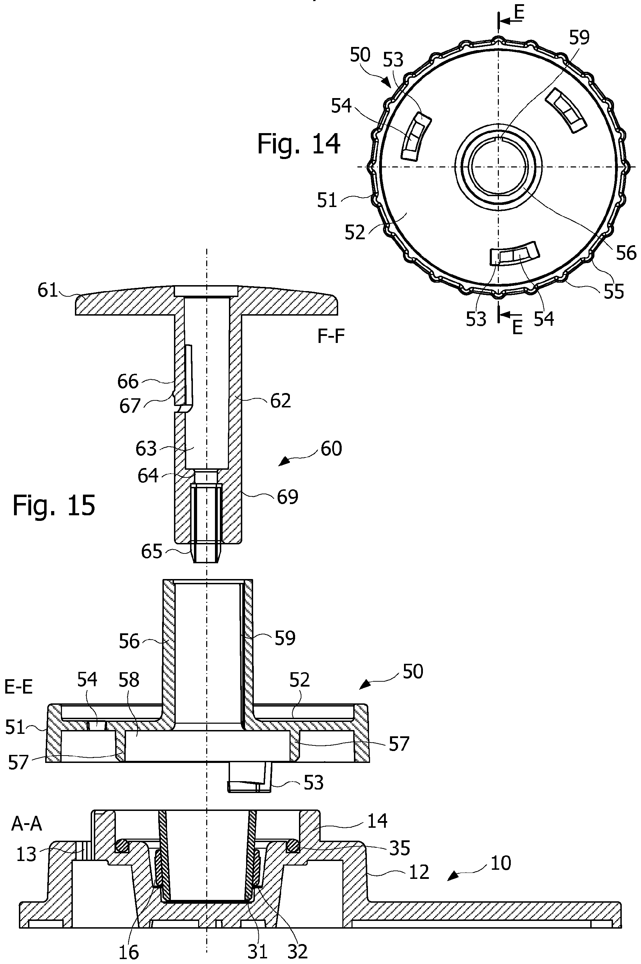

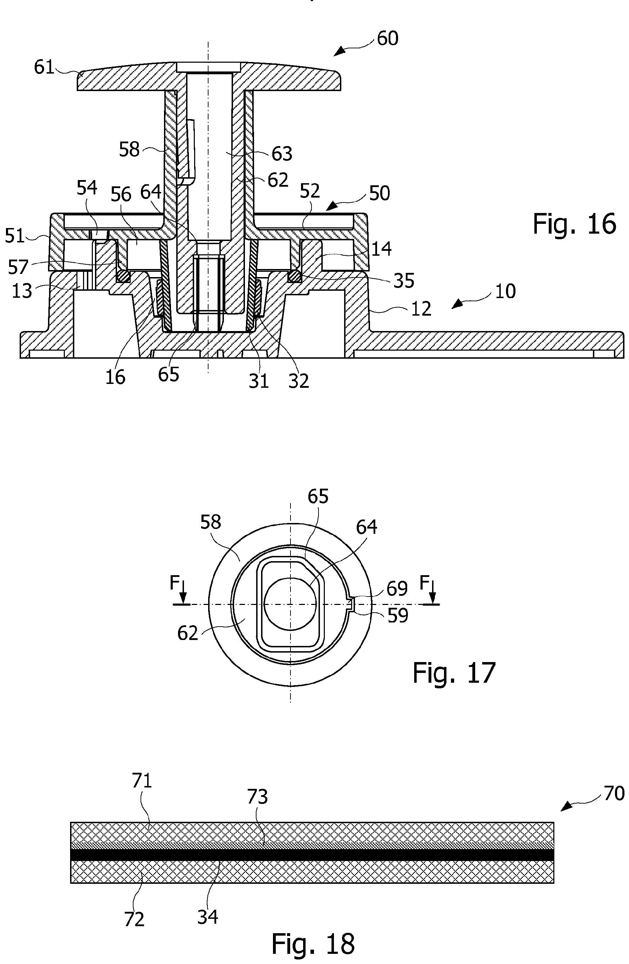

La figure 14 représente un couvercle 50 d'extraction de l'implant, selon un mode de réalisation. Les figures 15 et 16 représentent le dispositif de manipulation 10 muni du couvercle d'extraction 50, et d'un dispositif de découpe 60, selon un mode de réalisation. Une fois arrivé au site de transplantation, le couvercle de transport 40 est retiré et remplacé par le couvercle d'extraction 50. Le dispositif de découpe 60 est inséré dans le couvercle d'extraction 50 pour prélever l'implant en découpant un morceau de la membrane supportant la culture de cellules à implanter et recouverte des deux côtés de la couche de gel.

Le couvercle d'extraction 50 présente une forme similaire à celle du couvercle de préparation 20, dans lequel l'extension tubulaire inférieure 26 est remplacée par une extension tubulaire supérieure 56 formant un conduit tubulaire traversant le couvercle 50, permettant le passage du dispositif de découpe 60. Ainsi, le couvercle d'extraction 50 comprend un plateau supérieur 52 de forme annulaire, prolongé à l'extérieur par une couronne extérieure 51. Le couvercle d'extraction 50 comprend également des moyens de fixation 53 s'étendant du côté inférieur du couvercle et conformés pour coopérer avec les moyens de fixation 13 du dispositif de manipulation 10. Le couvercle d'extraction 50 comprend également une nervure annulaire 57 s'étendant du côté inférieur du couvercle, et prévue pour coopérer avec le joint torique 35 disposé dans la gorge annulaire 15 formée dans le dispositif de manipulation 10, pour assurer l'étanchéité de la fermeture du logement 16. La nervure annulaire 57 délimite un logement 58 pour recevoir la partie supérieure du dispositif support 30.

Dans l'exemple des figures 14 à 16, les moyens de fixation 53 présentent la forme de crochets prévus pour s'engager simultanément dans la partie la plus large des ouvertures 13. Les crochets 53 comportent une partie axiale prolongée par une partie radiale prévue pour se bloquer dans la partie la plus étroite des ouvertures 13 par un mouvement de rotation du couvercle d'extraction 50 autour de son axe par rapport au dispositif de manipulation 10. Pour faciliter l'entrainement en rotation par la main du couvercle 50, la couronne extérieure 51 peut être munie de crans 55. Des ouvertures 54 peuvent être formées dans le plateau supérieur 52, à l'aplomb des parties radiales des crochets 53 pour des raisons de fabrication.

L'extension tubulaire supérieure 56 est prévue pour recevoir le dispositif de découpe 60. Le dispositif de découpe 60 comprend un plateau supérieur 61 en forme de disque dont la face inférieure est prolongée axialement par une extension tubulaire 62 munie d'un conduit central axial traversant 63 comportant un épaulement 64 contre lequel vient en appui un outil de découpe 65 engagé par l'ouverture inférieure du conduit axial 63.

Dans l'exemple de la figure 15, l'extension tubulaire supérieure 56 comprend une nervure axiale 69 prévue pour coopérer avec une rainure formée dans la paroi interne du conduit axial 63, de manière à forcer l'orientation angulaire du dispositif de découpe 60 par rapport au couvercle 50 suivant un angle précis. L'extension tubulaire 62 peut comprendre un ergot 67 formé au voisinage de l'extrémité libre d'une languette axiale 66. L'ergot 67 est prévu pour qu'en fin de course du dispositif de découpe 60 dans le couvercle 50, l'ergot 67 s'efface en exerçant un frottement sur l'extension tubulaire supérieure 56. Ainsi l'opérateur est obligé d'appuyer sur le plateau 61 jusqu'à ce qu'il soit en butée contre le bord supérieur de l'extension tubulaire supérieure 56, ce qui permet d'assurer que l'outil de découpe 65 a bien traversé la membrane 34 et ses deux couches de gel. En effet, comme cela apparaît sur la figure 16, l'outil de découpe 65 arrive au contact du fond du logement 16 lorsque le plateau 61 arrive en butée contre le bord supérieur de l'extension tubulaire supérieure 56.

La figure 17 représente plus en détail l'extrémité inférieure du dispositif de découpe 60, et en particulier la nervure radiale 69 qui coopère avec la rainure radiale 59 du couvercle 50 et la forme de l'outil de découpe 65. L'outil de découpe 65 présente la forme d'un emporte-pièce de forme tubulaire, avec un bord inférieur tranchant pour découper un morceau de la membrane 35 supportant la culture de cellules à implanter recouverte des deux côtés d'une couche de gel. L'outil de découpe 65 permet de découper un morceau de membrane ayant une forme non symétrique, de manière à pouvoir déterminer sur quelle face de l'implant se trouve la culture de cellule. Cette disposition permet de disposer l'implant correctement dans le corps du patient, en particulier s'il doit être placé sur une surface. Le couvercle d'extraction 50 assure ainsi des fonctions de guidage et d'orientation de l'outil de découpe 65.

Dans l'exemple de la figure 17, l'outil de découpe 65 permet de découper un morceau d'implant sensiblement en forme de rectangle dont un coin a été retiré pour lui donner une forme non symétrique. L'orientation de l'outil de découpe 65 obtenu grâce à la nervure radiale 69 qui coopère avec la rainure radiale 59 du couvercle 50, peut être définie de manière à ce que la lame de l'outil de découpe soit à distance de l'orifice de remplissage 18.

La figure 18 représente l'implant 70 extrait de la membrane, obtenu après l'opération de découpe à l'aide du dispositif de découpe 60. L'implant 70 comprend les deux couches de gel 71, 72 couvrant d'un côté la membrane 34 et de l'autre la culture de cellules 73 formée sur la membrane 34.

Lorsqu'on retire l'outil de découpe 60 du couvercle d'extraction 50, il peut arriver que l'implant 70 ainsi découpé reste dans l'outil de découpe 50. Comme le dispositif de découpe 60 et l'outil de découpe 65 présentent une forme tubulaire, il est possible d'introduire une tige par l'ouverture supérieure du conduit axial 63 au travers du plateau 61, pour pousser l'implant 70 hors de l'outil de découpe 65, la tige présentant un diamètre inférieur à celui de l'épaulement 64. Si l'implant 70 reste dans le logement 16, il peut être extrait de ce dernier à l'aide d'une spatule.

Pour un implant de cellules d'épithélium pigmentaire de rétine humaine, l'outil de découpe 65 permet de découper un morceau de membrane 34 de 2 à 6 mm par 1,2 à 4 mm, par exemple de 5 par 3 mm ou de 2,5 par 1,5 mm, soit une surface comprise entre 10 et 20 mm², par exemple 15 mm² (ces valeurs pouvant varier dans des proportions de 20%). Ces dimensions permettent d'extraire de la membrane 34 un implant comportant quelques centaines de milliers de cellules.

L'implant 70 ainsi obtenu présente une épaisseur et des dimensions parfaitement maitrisées. Il peut être observé que durant toute la préparation et le transport de l'implant 70, jusqu'à son extraction au moment de réaliser la transplantation, la membrane 34 reste fixée à son support 30. On évite ainsi tout risque de détérioration de la culture de cellules. Les périodes où le boitier renfermant la membrane associée à son support est ouvert sont limitées aux étapes de changement de couvercle et à l'étape de découpe de l'implant. Les risques de contamination de l'implant restent donc limités. En outre, le fait de maintenir la membrane 34 fixée à son support 30 jusqu'à la découpe de l'implant 70, et de prévoir une forme dissymétrique pour l'implant, permet de conserver la mémoire de la face de la membrane supportant la culture de cellules jusqu'à l'opération de transplantation. Par ailleurs, les seuls outils qui peuvent être nécessaires en plus des éléments précédemment décrits sont limités à une tige poussoir pour, le cas échéant, faire sortir l'implant de l'outil de découpe 65, la seringue de transplantation et une spatule pour attraper et disposer l'implant dans la seringue.

Il apparaîtra clairement à l'homme de l'art que la présente invention est susceptible de diverses variantes de réalisation et diverses applications. En particulier, l'invention n'est pas limitée à un boitier dans lequel les couches de gel sont définies entre un logement et un couvercle. En effet, il peut être prévu d'utiliser un boitier muni d'une fente dans laquelle est insérée la membrane séparée de son support, la fente présentant une largeur calibrée.

Il n'est pas non plus nécessaire de disposer d'un couvercle de transport. En effet, il peut être prévu de disposer le boitier utilisé pour le moulage des couches de gel (dispositif de manipulation 10 + le couvercle de préparation 20) dans un sachet étanche, les couches de gel ne pouvant s'écouler par les orifices 18, 28 tant que le boitier est maintenu à une température suffisamment faible. Il peut également être envisagé de fermer de manière étanche les orifices 18, 28.

Il n'est pas non plus nécessaire de disposer d'un couvercle d'extraction. En effet, le morceau de membrane à implanter peut être découpé et extrait directement du logement 16 à l'aide d'un emporte-pièce. Il peut également être découpé par exemple au moyen d'un scalpel ou de ciseaux.

Il n'est pas non plus nécessaire que l'implant présente une forme dissymétrique. D'autres moyens peuvent être aisément imaginés pour repérer la face de la membrane supportant la culture de cellules. Ainsi, par exemple, la couche de gel formée sur l'une des deux faces de la membrane peut être colorée au moyen d'un colorant mélangé au liquide injecté par l'un des deux orifices 18, 28.

Il est également à noter que la préparation de l'implant à l'aide du couvercle de préparation 20 et l'utilisation de l'implant après découpe de la membrane peuvent être effectuées dans des sites éloignés géographiquement. Par conséquent, l'étape d'extraction de l'implant par découpe de la membrane peut ne pas être effectuée.

Il peut être également prévu de remplir simultanément les espaces supérieur 38 et inférieur 39 de chaque côté de la membrane 34, par exemple en prévoyant des passages latéraux dans le rebord 27a (ou en supprimant ce rebord) et dans l'élément tubulaire 31 du support 30. Dans ce cas, un seul des deux orifices 18, 28 est nécessaire.

Par ailleurs, tout autre moyen de fixation des couvercles 20, 40, 50 sur le dispositif de préparation 10 que celui décrit peut être envisagé. Ainsi, les couvercles 20, 40, 50 peuvent également se visser sur le dispositif de préparation 10 ou être fixés à ce dernier par clipsage.

L'invention peut s'appliquer à d'autres implants que des implants de cellules d'épithélium pigmentaire rétinien. En effet, l'invention peut s'appliquer à la transplantation de cellules de la rétine de sous-types variés et utilisés seuls ou en association (par exemple pour la rétine: des photorécepteurs, des cellules ganglionnaires, des cellules bipolaires, des cellules amacrines, des cellules de l'épithélium pigmentaire rétinien, des cellules endothéliales). Ces cellules peuvent être associées en monocouches ou en couches pluristratifiées de façon à reconstruire une rétine artificielle. Il peut s'agir également notamment de cellules de la cornée ou de tout autre type de cellules épithéliales. Plus généralement, l'invention peut s'appliquer à tout type d'épithélium ou de culture de cellules en monocouche sur une membrane biologique ou synthétique.

## Revendications

1. Procédé de préparation d'un implant (70) comprenant une culture de cellules (73) sur une membrane (34), le procédé comprenant des étapes consistant à:
fixer la membrane sur un support (30),
disposer le support dans un logement (16) d'un boitier (10, 20) ménageant deux espaces (38, 39) ayant des hauteurs ajustées, au-dessus et en dessous de la membrane,
injecter dans les espaces un liquide apte à se transformer en gel à une température de transport inférieure à une température d'injection du liquide, et
porter le boitier à la température de transport, de manière à former un implant comprenant la membrane et deux couches de gel (71, 72) ayant des épaisseurs ajustées, respectivement sur deux faces opposées de la membrane.

2. Procédé selon la revendication 1, comprenant une étape de découpe de l'implant (70) dans la membrane (34) et les deux couches de gel (71, 72), la membrane étant fixée au support (30) et disposée dans le logement (16).

3. Procédé selon l'une des revendications 1 et 2, comprenant une étape de fermeture du logement (16) du boitier (10) recevant la membrane par un couvercle d'extraction (50), le couvercle d'extraction étant configuré pour recevoir et guider un outil de découpe (60) configuré pour découper l'implant (70) dans la membrane (34) et les deux couches de gel (71, 72).

4. Procédé selon l'une des revendications 1 à 3, dans lequel l'implant (70) présente une forme dissymétrique.

5. Procédé selon l'une des revendications 1 à 4, dans lequel les espaces (38, 39) au-dessus et en dessous de la membrane (34) sont formés entre le fond du logement (16) et un couvercle de préparation (20) adapté à refermer le logement.

6. Procédé selon l'une des revendications 1 à 5, comprenant une étape de remplacement du couvercle de préparation (20) par un couvercle de transport (40) refermant le logement (16) de manière étanche, lorsque le liquide injecté dans le logement s'est polymérisé en gel.

7. Procédé selon l'une des revendications 1 à 6, dans lequel les liquides injectés dans les espaces supérieur (38) et inférieur (39) sont différents, de manière à ce que la couche de gel supérieure (71) soit moins rigide que la couche de gel inférieure (72).

8. Procédé selon l'une des revendications 1 à 7, dans lequel les cellules disposées sur la membrane sont dérivées de cellules souches pluripotentes, de cellules souches adultes multipotentes ou bien sont issues de cultures primaires ou de lignées cellulaires, ou bien correspondent à un type cellulaire de la zone d'implantation, ou bien sont des cellules épithéliales simples ou de différents sous-types, tels que des photorécepteurs, des cellules ganglionnaires, des cellules bipolaires, des cellules amacrines, des cellules de l'épithélium pigmentaire rétinien, des cellules endothéliales.

9. Dispositif de préparation d'un implant à partir d'une culture de cellules sur une membrane, le dispositif comprenant :
un support (30) maintenant la membrane (34),
un boitier (10, 20) comportant un logement (16) pour recevoir le support associé à la membrane, le logement ménageant deux espaces (38, 39) ayant des hauteurs ajustées, au-dessus et en dessous de la membrane, et
un orifice de remplissage (18, 28) pour injecter un liquide dans les deux espaces, le liquide étant apte à se transformer en gel à une température de transport inférieure à une température d'injection du liquide, le dispositif étant configuré pour mettre en œuvre le procédé selon l'une des revendications 1 à 8.

10. Dispositif selon la revendication 9, comprenant un couvercle de préparation (20) configuré pour refermer le logement (16) avec le support (30), le couvercle de préparation délimitant avec la membrane (34) l'espace supérieur (38), l'espace inférieur (39) étant délimité par le fond du logement et la membrane, le couvercle de préparation comprenant un orifice de remplissage (28) débouchant dans l'espace supérieur, le boitier comprenant un orifice de remplissage (18) débouchant dans l'espace inférieur.

11. Dispositif selon la revendication 9 ou 10, comprenant un couvercle de transport (40) prévu pour refermer le logement (16) de manière étanche.

12. Dispositif selon l'une des revendications 9 à 11, comprenant un couvercle d'extraction (50) prévu pour refermer le logement (16) et comportant un conduit (56) pour le passage d'un outil de découpe (60) configuré pour découper un morceau de la membrane (34) incluant des cellules cultivées, le morceau de membrane découpé formant l'implant (70),

13. Dispositif selon l'une des revendications 9 à 12, comprenant au moins l'une des caractéristiques suivantes :
les deux espaces (38, 39) présentent une hauteur comprise entre 0,05 et 0,2 mm, ou bien entre 0,13 et 0,17 mm,
l'implant (70) présente une surface comprise entre 10 et 20 mm².
la membrane (34) est une membrane amniotique, ou une membrane de Bruch, ou une membrane basale, ou une matrice de fibres protéiques telles que des fibres de collagène et/ou de fibrine ou une membrane en un matériau synthétique biocompatible, biodégradable ou non,
le liquide se transformant en gel est un hydrogel naturel ou synthétique biocompatible, qui est liquide à la température intérieure du corps humain,
le support (30) de membrane présente une forme tronconique comportant une grande ouverture et une petite ouverture, la membrane étant fixée sur le support de manière à obturer la petite ouverture, la culture de cellules étant réalisée sur une face de la membrane disposée à l'intérieur du support,
l'outil de découpe (60) est configuré pour découper une morceau de membrane de forme dissymétrique.

14. Utilisation du dispositif selon l'une des revendications 9 à 13, pour réaliser un implant de cellules cultivées sur une membrane (34).

## Patentansprüche

1. Verfahren zur Herstellung eines Implantats (70), das eine Zellkultur (73) auf einer Membran (34) umfasst, wobei das Verfahren die folgenden Schritte umfasst:
Befestigen der Membran auf einem Träger (30),
Anordnen des Trägers in einer Aufnahme (16) eines Gehäuses (10, 20) mit Ausbildung von zwei Zwischenräumen (38, 39) mit angepassten Höhen oberhalb und unterhalb der Membran,
Einspritzen einer Flüssigkeit in die Zwischenräume, die in der Lage ist, sich bei einer Transporttemperatur, die niedriger als eine Einspritztemperatur der Flüssigkeit ist, in Gel zu verwandeln, und
Bringen des Gehäuses auf die Transporttemperatur, um so ein Implantat zu bilden, das die Membran und zwei Gelschichten (71, 72) mit angepassten Dicken auf zwei entsprechenden gegenüberliegenden Seiten der Membran umfasst.

2. Verfahren nach Anspruch 1, welches einen Schritt des Zuschneidens des Implantats (70) in der Membran (34) und den zwei Gelschichten (71, 72) umfasst, wobei die Membran auf dem Träger (30) befestigt und in der Aufnahme (16) angeordnet ist.

3. Verfahren nach einem der Ansprüche 1 und 2, welches einen Schritt des Schließens der Aufnahme (16) des Gehäuses (10), welche die Membran aufnimmt, mit einem Extraktionsdeckel (50) umfasst, wobei der Extraktionsdeckel dafür ausgelegt ist, ein Schneidwerkzeug (60) aufzunehmen und zu führen, das dafür ausgelegt ist, das Implantat (70) in der Membran (34) und den zwei Gelschichten (71, 72) zuzuschneiden.

4. Verfahren nach einem der Ansprüche 1 bis 3, wobei das Implantat (70) eine asymmetrische Form aufweist.

5. Verfahren nach einem der Ansprüche 1 bis 4, wobei die Zwischenräume (38, 39) oberhalb und unterhalb der Membran (34) zwischen dem Boden der Aufnahme (16) und einem Präparationsdeckel (20), der dafür eingerichtet ist, die Aufnahme zu verschließen, ausgebildet sind.

6. Verfahren nach einem der Ansprüche 1 bis 5, welches einen Schritt des Ersetzens des Präparationsdeckels (20) durch einen Transportdeckel (40), der die Aufnahme (16) dicht verschließt, umfasst, wenn die in die Aufnahme eingespritzte Flüssigkeit sich zu Gel polymerisiert hat.

7. Verfahren nach einem der Ansprüche 1 bis 6, wobei die in den oberen (38) und den unteren (39) Zwischenraum eingespritzten Flüssigkeiten verschieden sind, derart, dass die obere Gelschicht (71) weniger starr als die untere Gelschicht (72) ist.

8. Verfahren nach einem der Ansprüche 1 bis 7, wobei die auf der Membran angeordneten Zellen Derivate von pluripotenten Stammzellen oder von multipotenten adulten Stammzellen sind, oder auch aus Primärkulturen oder aus Zelllinien hervorgegangen sind, oder auch einem Zelltyp der Implantationszone entsprechen, oder auch einfache Epithelzellen sind, oder von verschiedenen Untertypen sind, wie etwa Photorezeptoren, Ganglienzellen, Bipolarzellen, Amakrinzellen, Zellen des retinalen Pigmentepithels, Endothelzellen.

9. Vorrichtung zur Herstellung eines Implantats aus einer Zellkultur auf einer Membran, wobei die Vorrichtung umfasst:
einen Träger (30), der die Membran (34) hält,
ein Gehäuse (10, 20), das eine Aufnahme (16) zum Aufnehmen des mit der Membran verbundenen Trägers aufweist, wobei die Aufnahme zwei Zwischenräume (38, 39) mit angepassten Höhen oberhalb und unterhalb der Membran ausbildet, und
eine Einfüllöffnung (18, 28) zum Einspritzen einer Flüssigkeit in die zwei Zwischenräume, wobei die Flüssigkeit in der Lage ist, sich bei einer Transporttemperatur, die niedriger als eine Einspritztemperatur der Flüssigkeit ist, in Gel zu verwandeln, wobei die Vorrichtung dafür ausgelegt ist, das Verfahren nach einem der Ansprüche 1 bis 8 durchzuführen.

10. Vorrichtung nach Anspruch 9, welche einen Präparationsdeckel (20) umfasst, der dafür ausgelegt ist, die Aufnahme (16) mit dem Träger (30) zu verschließen, wobei der Präparationsdeckel mit der Membran (34) den oberen Zwischenraum (38) begrenzt, wobei der untere Zwischenraum (39) vom Boden der Aufnahme und der Membran begrenzt wird, wobei der Präparationsdeckel eine Einfüllöffnung (28) umfasst, die in den oberen Zwischenraum mündet, wobei das Gehäuse eine Einfüllöffnung (18) umfasst, die in den unteren Zwischenraum mündet.

11. Vorrichtung nach Anspruch 9 oder 10, welche einen Transportdeckel (40) umfasst, der dafür vorgesehen ist, die Aufnahme (16) dicht zu verschließen.

12. Vorrichtung nach einem der Ansprüche 9 bis 11, welche einen Extraktionsdeckel (50) umfasst, der dafür vorgesehen ist, die Aufnahme (16) zu verschließen, und einen Kanal (56) zum Durchlassen eines Schneidwerkzeugs (60) aufweist, das dafür ausgelegt ist, ein Stück der Membran (34), das kultivierte Zellen aufweist, auszuschneiden, wobei das ausgeschnittene Membranstück das Implantat (70) bildet.

13. Vorrichtung nach einem der Ansprüche 9 bis 12, welche wenigstens eines der folgenden Merkmale umfasst:
Die zwei Zwischenräume (38, 39) weisen eine Höhe zwischen 0,05 und 0,2 mm auf, oder auch zwischen 0,13 und 0,17 mm,
das Implantat (70) weist eine Fläche zwischen 10 und 20 mm² auf,
die Membran (34) ist eine amniotische Membran oder eine Bruch-Membran oder eine Basalmembran oder eine Matrix aus Proteinfasern, wie etwa Kollagen- und/oder Fibrinfasern, oder eine Membran aus einem biokompatiblen synthetischen Material, das biologisch abbaubar ist oder nicht,
die Flüssigkeit, die sich in Gel verwandelt, ist ein natürliches oder synthetisches, biokompatibles Hydrogel, welches bei der Innentemperatur des menschlichen Körpers flüssig ist,
der Träger (30) der Membran weist eine kegelstumpfförmige Gestalt auf, die eine große Öffnung und eine kleine Öffnung aufweist, wobei die Membran derart auf dem Träger befestigt ist, dass sie die kleine Öffnung verschließt, wobei die Zellkultur auf einer Seite der Membran hergestellt ist, die im Inneren des Trägers angeordnet ist,
das Schneidwerkzeug (60) ist dafür ausgelegt, ein Membranstück von asymmetrischer Form auszuschneiden.

14. Verwendung der Vorrichtung nach einem der Ansprüche 9 bis 13, um ein Implantat mit kultivierten Zellen auf einer Membran (34) herzustellen.

## Claims

1. A method of preparing an implant (70) comprising a culture of cells (73) on a membrane (34), the method comprising the steps of:
fixing the membrane on a support (30), disposing the support in a housing recess (16) of a housing (10, 20) leaving two spaces (38, 39) having adjusted heights, above and below the membrane,
injecting into the spaces a liquid capable of transforming into gel at a temperature of transport below an injection temperature of the liquid, and
bringing the housing to the transport temperature, so as to form an implant comprising the membrane and two layers of gel (71, 72) having adjusted thicknesses, respectively on two opposite faces of the membrane.

2. Method according to claim 1, comprising a step of cutting the implant (70) in the membrane (34) and the two layers of gel (71, 72), the membrane being fixed to the support (30) and disposed in the housing recess (16).

3. Method according to one of claims 1 and 2, comprising a step of closing the housing recess (16) of the housing (10) receiving the membrane by an extraction cover (50), the extraction cover being configured to receive and guide a cutting tool (60) configured to cut the implant (70) in the membrane (34) and the two layers of gel (71, 72).

4. Method according to one of claims 1 to 3, wherein the implant (70) has an asymmetrical shape.

5. Method according to one of claims 1 to 4, wherein the spaces (38, 39) above and below the membrane (34) are formed between the bottom of the housing recess (16) and a preparation cover (20) suitable for closing the housing recess.

6. Method according to one of claims 1 to 5, comprising a step of replacing the preparation cover (20) by a transport cover (40) closing the housing recess (16) in a sealed manner, when the liquid injected into the housing recess is gel polymerized.

7. Method according to one of claims 1 to 6, in which the liquids injected into the upper (38) and lower (39) spaces are different, so that the upper gel layer (71) is less rigid than the lower gel layer(72).

8. Method according to one of claims 1 to 7, in which the cells arranged on the membrane are derived from pluripotent stem cells, from multipotent adult stem cells or else are derived from primary cultures or cell lines, or else correspond to a cell type of the implantation area, or are simple epithelial cells of different subtypes, such as photoreceptors, ganglion cells, bipolar cells, amacrine cells, retinal pigment epithelial cells, endothelial cells .

9. Device for preparing an implant from a cell culture on a membrane, the device comprising:
a support (30) holding the membrane (34), a housing (10, 20) comprising a housing recess (16) for receiving the support associated with the membrane, the housing providing two spaces (38, 39) having adjusted heights, above and below the membrane, and
a filling orifice (18, 28) for injecting liquid into the two spaces, the liquid being capable of transforming into a gel at a transport temperature below an injection temperature of the liquid, the device being configured to implement the method according to one of claims 1 to 8.

10. Device according to claim 9, comprising a preparation cover (20) configured to close the housing recess (16) with the support (30), the preparation cover delimiting with the membrane (34) the upper space (38), the lower space (39) being delimited by the bottom of the housing recess and the membrane, the preparation cover comprising a filling orifice (28) opening into the upper space, the housing comprising a filling orifice (18) opening into the space inferior.

11. Device according to claim 9 or 10, comprising a transport cover (40) provided to close the housing (16) in a sealed manner.

12. Device according to one of claims 9 to 11, comprising an extraction cover (50) provided to close the housing recess (16) and comprising a tubular extension (56) for the passage of a cutting tool (60) configured to cut a piece of the membrane (34) including cultured cells, the cut piece of membrane forming the implant (70).

13. Device according to one of Claims 9 to 12, comprising at least one of the following characteristics:
the two spaces (38, 39) have a height of between 0.05 and 0.2 mm, or else between 0.13 and 0.17 mm,
the implant (70) has an area of between 10 and 20 mm²
the membrane (34) is an amniotic membrane, or a Bruch's membrane, or a basal membrane, or a matrix of protein fibers such as collagen and / or fibrin fibers or a membrane made of a biocompatible synthetic material, biodegradable or not,
the liquid turning into gel is a natural or synthetic biocompatible hydrogel, which is liquid at the internal temperature of the human body,
the membrane support (30) has a truncated cone shape with a large opening and a small opening, the membrane being attached on the support so as to close the small opening, the cell culture being carried out on one face of the membrane disposed inside the support, the cutting tool (60) is configured to cut a piece of membrane of asymmetric shape .

14. Use of the device according to one of claims 9 to 13, for producing an implant of cells cultured on a membrane (34).
